# EUROPEAN PATENT APPLICATION

(11) **EP 4 079 262 A1**
(43) Date of publication of application: **26.10.2022**
(21) Application number: 20908261.9
(22) Date of filing: 22.12.2020
(51) Int. Cl.: A61F 2/32

(54) **FLEXIBLE HIP JOINT STEM AND HIP JOINT PROSTHESIS USING SAME**

(30) Priority: 23.12.2019 CN 201911344107
(71) Applicant: Zhu, Hongwen, Tianjin 300071 (CN)
(72) Inventor: ZHU, Hongwen, Tianjin 300071 (CN); HUANG, Guofu, Tianjin 300071 (CN); DONG, Ronghua, Tianjin 300071 (CN); ZHU, Tianmou, Tianjin 300071 (CN)
(74) Representative: Sun, Yiming
(86) International application number: PCT/CN2020/138366
(87) International publication number: WO 2021/129620

(57) **Abstract**

The present invention discloses a flexible hip joint stem. The flexible hip joint stem includes a shoulder body connecting piece, a flexible stem body, a stem tail, and a center regulating rod. The flexible stem body includes an inner-layer cylinder and an outer-layer cylinder. The inner-layer cylinder and the outer-layer cylinder are both composed of spiral springs, where the spring wire width of the spiral springs is basically equal to the screw pitch. The regulating rod passes through the shoulder body connecting piece and the flexible stem body, and is fixed with the stem tail by screwing the threads, which allows the inner-layer cylinder and the outer-layer cylinder to produce a certain degree of elastic deformation, limits the range of the elastic deformation, and enables the flexible hip joint stem to have sufficient supporting strength as a whole.

## Description

### TECHNICAL FIELD

The present invention relates to an implant in the human body applied in orthopaedics, in particular, to a flexible hip joint stem and a flexible hip joint prosthesis using the same.

### BACKGROUND ART

In recent years, the orthopedic application of artificial joints in the treatment of joint injuries has been widely used, and the therapeutic effect has been recognized by numerous doctors and patients. The artificial joint prostheses commonly used now have a history of several decades. In particular, further changes are made in materials, mechanics, morphology, and process requirements. However, due to various uncertain factors of artificial joints and biological tissues, there are still some unsatisfactory problems in the user process; in particular, further research and improvement are required for artificial prostheses and physiological functions to make the prosthesis meet the physiological requirements of histology, especially the dynamic requirements of femoral function, so as to effectively reduce some complications caused by prosthesis factors after hip joint surgeries, especially the loosening and sinking of the prosthesis. During the follow-up consultation, it was found that there were also some cases of stem fracture. Finding ways to reduce complications is the key to continuously improving the service life of the stem body.

Specifically, when the existing hip joint prosthesis is mounted in a human body, the artificial hip joint prosthesis imitates the structure of the human hip joint, including a prosthetic metal cup for replacing the acetabulum and a spherical end for replacing the femoral head, wherein the spherical end is fixed on the hip joint stem, a bolt is provided at the lower end of the hip joint stem, and the hip joint stem is fixed on the human femur by inserting the bolt into the femoral medullary cavity; a rotating mechanism is formed by the spherical end with the acetabulum or prosthetic metal cup to realize bending and movement of the femur.

In the existing artificial hip replacement surgery, it is possible to choose a bowl-shaped acetabulum for replacement or a femoral stem with a spherical end for replacement according to the specific injury and condition, or to choose both at the same time for replacement. However, after a period of time, the tightness between the hip joint stem and the femur on which it is located gradually decreases, and the two become loose. Under the action of body pressure, the hip joint stem and the bolt on it will sink further, causing the two legs of the patient to have different lengths, which will further affect the normal physiological function of the hip joint.

The bolts on the existing hip joint stems are all straight rods. In order to insert the bolts into the medullary cavity of the femur, the doctor needs to drill a deep hole in the middle of the patient's medullary cavity, which is naturally a vertical hole. However, the human femur is physiologically curved, not truly straight rod-shaped. Thus, when preparing holes required for mounting existing artificial hip joints, it is necessary to destroy the cancellous bone to reduce the overall strength of the femur. Moreover, the straight rod-shaped structure mainly focuses on the inner condyle regarding the bearing capacity of the vertical force.

In order to enhance the strength of the femur after surgery, the most ideal treatment is to provide a hole along the natural physiological curvature of the femoral medullary cavity and then insert the hip joint stem into the femoral medullary cavity. However, the existing hip joint prosthesis cannot be securely mounted in such a femoral medullary cavity.

In view of the above problems, the inventors have conducted in-depth research on the existing hip joint prostheses, hoping to design a new flexible hip joint stem and a new flexible hip joint prosthesis that can solve the above problems.

### SUMMARY OF THE INVENTION

In order to overcome the above problems, the inventors have conducted intensive research and devised a flexible hip joint stem. The flexible hip joint stem includes a shoulder body connecting piece, a flexible stem body, a stem tail, and a center regulating rod. The flexible stem body includes an inner-layer cylinder and an outer-layer cylinder. The inner-layer cylinder and the outer-layer cylinder are both composed of spiral springs, where the spring wire width of the spiral springs is basically equal to the screw pitch. The regulating rod passes through the shoulder body connecting piece and the flexible stem body and is fixed with the stem tail by screwing the threads, which allows the inner-layer cylinder and the outer-layer cylinder to produce a certain degree of elastic deformation, limits the range of the elastic deformation, and enables the flexible hip joint stem to have sufficient supporting strength as a whole.

Specifically, the object of the present invention is to provide a flexible hip joint stem that is used for being inserted into the femoral medullary cavity on the femoral body. The flexible hip joint stem includes a flexible stem body 22 with lateral deformation capability, wherein the flexible stem body 22 can be deformed along the physiological arc of a human femoral medullary cavity and is in close contact with the femoral medullary cavity. The flexible hip joint stem is mounted on the femoral body through the cooperation of the flexible stem body 22 with the femoral medullary cavity.

The object of the present invention is to further provide flexible hip joint prosthesis, including a femoral stem 1 and a flexible hip joint stem 2, wherein an artificial spherical end 3 is provided on the femoral stem 1.

The beneficial effects of the present invention include the following:
(1) the flexible hip joint stem according to the present invention can be inserted into the femoral body along the physiological arc of the femoral medullary cavity, so as to be in close contact with the femoral body and to improve the connection stability between the flexible hip joint stem and the femoral body and the strength of the femoral shaft by destroying the femoral body as little as possible;
(2) in the flexible hip joint stem according to the present invention, a center regulating rod is provided in the middle of the flexible stem body, which allows elastic deformation of the flexible stem body, limits its amount of deformation, and ensures sufficient connection/supporting strength;
(3) in the flexible hip joint prosthesis according to the present invention, a force-bearing portion capable of abutting on the inner side of the cortex of the greater trochanter is provided on the side of the femoral stem, thereby increasing the longitudinal load of the femoral stem; filling the force-bearing portion with cancellous bone, bone strips or stem cells will facilitate the integral growth of the femoral stem and the greater trochanter, and further improve the stability of the femoral stem while providing support and fixation force for the femoral stem in another direction; and
(4) a buffer device is provided in the flexible hip joint prosthesis according to the present invention, wherein the buffer device is mounted inside the artificial spherical end to further enhance the effect of buffering and shock absorption.

### BRIEF DESCRIPTION OF DRAWINGS

FIG. 1 illustrates a schematic diagram of the overall structure of a flexible hip joint stem according to a preferred embodiment of the present invention;

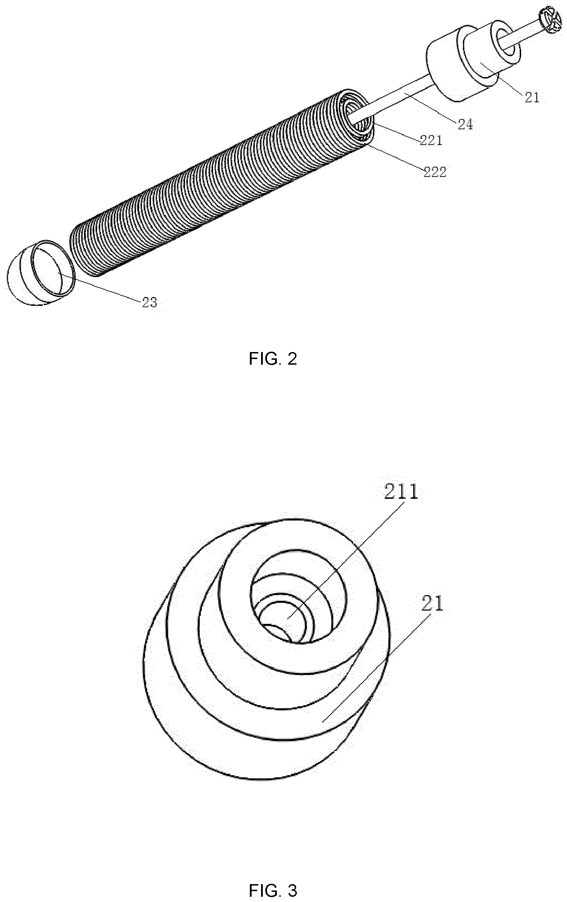

FIG. 2 illustrates an exploded view of a flexible hip joint stem according to a preferred embodiment of the present invention;
FIG. 3 illustrates a schematic structural diagram of a shoulder body connecting piece of a flexible hip joint stem according to a preferred embodiment of the present invention;
FIG. 4 illustrates a schematic structural diagram of a flexible stem body of a flexible hip joint stem according to a preferred embodiment of the present invention;
FIG. 5 illustrates a schematic structural diagram of a stem tail of a flexible hip joint stem according to a preferred embodiment of the present invention;

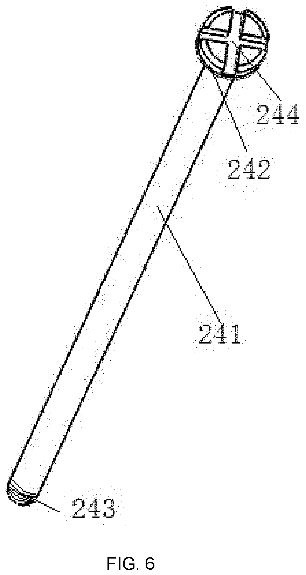

FIG. 6 illustrates a schematic structural diagram of a center regulating rod of a flexible hip joint stem according to a preferred embodiment of the present invention;
FIG. 7 illustrates a schematic structural diagram when the neck rod on the flexible hip joint prosthesis fully extends into the neck rod hole according to a preferred embodiment of the present invention;
FIG. 8 illustrates a schematic structural diagram when the neck rod of the flexible hip joint prosthesis is ejected to the maximum stroke by a buffer device according to a preferred embodiment of the present invention;
FIG. 9 illustrates a schematic structural diagram of an upper limit cavity and a limit protrusion of flexible hip joint prosthesis according to a preferred embodiment of the present invention;
FIG. 10 illustrates a schematic diagram of the overall structure of a flexible hip joint prosthesis according to a preferred embodiment of the present invention; and
FIG. 11 illustrates an exploded view of a femoral stem on flexible hip joint prosthesis according to a preferred embodiment of the present invention.

### DESCRIPTION OF THE REFERENCE SIGNS OF THE DRAWINGS

1 - femoral stem
11 - reserved space
12 - hole
13 - hard shell
14 - support ridge
2 - flexible hip joint stem
21 - shoulder body connecting piece
211 - through-hole
22 - flexible stem body
221 - inner-layer cylinder
222 - outer-layer cylinder
23 - stem tail
231 - circular platform
24 - center regulating rod
241 - long straight rod
242 - end head
243 - thread
245 - cross flower slot
3 - artificial spherical end
31 - buffer device
32 - neck rod hole
33 - buffer device accommodating cavity
34 - limit slot
4 - neck rod
41 - limit protrusion
42 - limit cavity
43 - compressed spring

### DETAILED DESCRIPTION OF EXEMPLIFIED EMBODIMENTS

The present invention will be further described in detail below through the accompanying drawings and examples. The characteristics and advantages of the present invention will become clearer through these descriptions.

The word "exemplary" specifically used herein means "serving as an example, embodiment or illustration". Any example described herein as "exemplary" is not necessarily to be construed as being superior to or better than other examples.

Although various aspects of the examples are shown in the drawings, the drawings are not necessarily drawn to scale unless otherwise indicated.

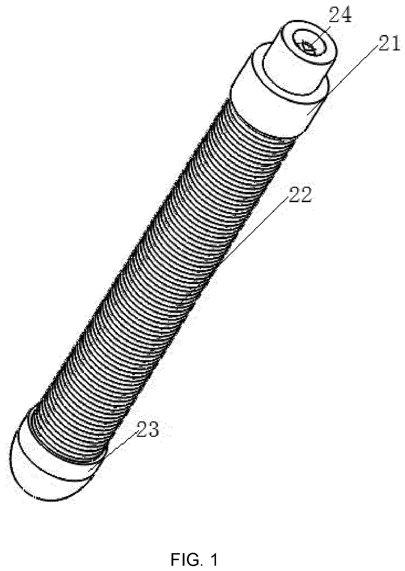

According to the flexible hip joint stem according to the present invention, the flexible hip joint stem includes a flexible stem body 22 with lateral deformation capability, as shown in FIG. 1. The flexible stem body 22 can be deformed in a femoral medullary cavity along the physiological arc of a human femoral medullary cavity and is in close contact with the femoral medullary cavity, whereby the flexible hip joint stem is mounted on the femoral body through the cooperation of the flexible stem body 22 with the femoral medullary cavity.

Preferably, the flexible hip joint stem 2 includes a shoulder connecting piece 21, a flexible stem body 22, a stem tail 23, and a center regulating rod 24, wherein the flexible stem body 22 includes an inner-layer cylinder 221 and an outer-layer cylinder 222.

The inner-layer cylinder 221 and the outer-layer cylinder 222 are both composed of spiral springs. In the absence of external force, the spring wire width of the inner-layer cylinder is basically equal to the screw pitch, i.e., any two adjacent coils of spring wires of the inner-layer cylinder 221 are attached to each other; the spring wire width of the spiral spring of the outer-layer cylinder is basically equal to the screw pitch, i.e., any two adjacent coils of spring wires of the outer-layer cylinder 222 are attached to each other.

Due to the above setting, both the inner-layer cylinder 221 and the outer-layer cylinder 222 are cylindrical and have a certain lateral deformation ability, i.e., they can be deformed along the physiological arc of the human femoral medullary cavity and are in close contact with the femoral medullary cavity to accomplish the fixation between the bolt 2 and the femur without causing excessive damage to the femoral medullary cavity.

Both the inner-layer cylinder 221 and the outer-layer cylinder 222 can be stretched and deformed outward, but cannot be deformed by pressing inward, and the amount of deformation caused by outward stretching and deformation is small. Due to this setting of the flexible stem body, there is a certain ability for stretching and deformation between the flexible hip joint stem and the femur, which leads to certain elastic deformation and buffering so that the femur installed with the flexible hip joint stem has a buffering and shock absorption ability similar to the naturally grown femur of the human body. In addition, when the flexible hip joint stem is installed in the femoral medullary cavity, the damage caused to the inner wall of the femoral medullary cavity, especially to the cortex, is very small, thereby prolonging the service life of the flexible hip joint stem.

The spring wire width refers to the cross-sectional dimension of the wire that is wound to form the spiral spring. When the cross-section of the wire is circular, the width/cross-sectional dimension of the spring wire specifically refers to the diameter of the circle. When the cross-section of the wire is rectangular, the width/cross-sectional dimension of the spring wire specifically refers to the length of the longest side of the rectangular cross-section.

In the present invention, preferably, the cross-sectional shape of the spring wire/wire is a rectangle, more preferably a square, and the edge of the spring wire/wire is provided with chamfered edges for a smooth transition.

Preferably, the wire is a metal material with biocompatibility.

In a preferred embodiment, the inner-layer cylinder 221 and the outer-layer cylinder 222 are formed by winding the same wire, that is, the inner-layer cylinder 221 is formed by winding a single wire, and then the outer-layer cylinder 222 is formed by further winding, where the wire is continuous during the wiring process. Alternatively, the outer-layer cylinder 222 is formed by winding a single wire, and then the inner-layer cylinder 221 is formed by further winding, where the wire is continuous during the winding process. By winding a single wire, the various acting forces received by the inner-layer cylinder 221 and the outer-layer cylinder 222 in the medullary cavity can be converted into elastic deformation of the spiral spring in time.

More preferably, the spiral spring of the inner-layer cylinder 221 rotates in an opposite direction to that of the outer-layer cylinder 222, so that the flexible stem body 22 has a limited amount of rebound recovery function and extremely high anti-fatigue properties.

The spiral spring of the inner-layer cylinder 221 rotates clockwise and the spiral spring of the outer-layer cylinder 222 rotates anticlockwise, or the spiral spring of the inner-layer cylinder 221 rotates anticlockwise and the spiral spring of the outer-layer cylinder 222 rotates clockwise.

In the present application, preferably, the spiral spring of the inner-layer cylinder 221 rotates anticlockwise and the spiral spring of the outer-layer cylinder 222 rotates clockwise.

In a preferred embodiment, the diameter size of the cylindrical shape formed by the inner-layer cylinder 221 and the outer-layer cylinder 222 gradually decreases from top to bottom, that is, the outer diameter size of the flexible stem body 22 gradually decreases from top to bottom, in order to adapt to the physiological size of the human femoral medullary cavity.

In a preferred embodiment, a certain gap is left between the inner-layer cylinder 221 and the outer-layer cylinder 222. Preferably, a gap of 2-4 mm is left between the inner-layer cylinder 221 and the outer-layer cylinder 222, so that there is no interference between the inner-layer cylinder 221 and the outer-layer cylinder 222, which can facilitate torsional deformation or bending deformation of the inner-layer cylinder 221 and the outer-layer cylinder 222.

In a preferred embodiment, the shoulder body connecting piece 21 is mounted at the top end of the flexible stem body 22, and the stem tail 23 is mounted at the bottom end of the flexible stem body 22.

Specifically, the upper end of the shoulder body connecting piece 21 is inserted into the femoral stem 1 and is fixedly connected with the femoral stem 1. The lower end of the shoulder body connecting piece 21 is provided with a space for accommodating the top of the flexible stem body 22. The top of the flexible stem body 22 is inserted into the shoulder body connecting piece 21. Preferably, the top of the flexible stem body 22 is in an interference fit with the shoulder body connecting piece 21.

In a preferred embodiment, the stem tail 23 is in a quasi-conical shape, a space for accommodating the bottom of the flexible stem body 22 is provided in the stem tail 23, and the bottom of the flexible stem body 22 is inserted into the stem tail 23. Preferably, the bottom of the flexible stem body 22 is in an interference fit with the stem tail 23.

The stem tail 23 in a quasi-conical shape can further reduce the damage to the inner wall of the femoral medullary cavity during the insertion of the flexible hip joint stem into the femoral medullary cavity, and can also adapt to the shape of the cavity provided in the femoral medullary cavity, thereby improving the connection strength between the flexible hip joint stem and the femoral medullary cavity.

In a preferred embodiment, the center regulating rod 24 passes through the shoulder body connecting piece 21 and the flexible stem body 22, and is fixed with the stem tail 23 by screwing the threads.

Preferably, the main body of the center regulating rod 24 is a long straight rod 241, the top of which is provided with an end head 242 and the lower end of which is engraved with threads 243.

A through-hole 211 is provided in the middle of the shoulder body connecting piece 21, and the through-hole 211 can allow the long straight rod 241 to pass through, but cannot allow the end head 242 to pass through.

The stem tail 23 is provided with a circular platform 231 engraved with an internal thread, and the center regulating rod 24 passes through the through-hole 211 on the shoulder body connecting piece 21 and the inner-layer cylinder 221 on the flexible stem body 22 in turn, and then rotate into the circular platform 231, so that the shoulder body connecting piece 21, the flexible stem body 22 and the stem tail 23 are consolidated into one body.

Preferably, the outer diameter of the long straight rod 241 is smaller than the inner diameter of the inner-layer cylinder 221. Moreover, a predetermined gap is left between the long straight rod 241 and the inner-layer cylinder 221, so as to allow the inner-layer cylinder 221 and the outer-layer cylinder 222 to produce a certain degree of elastic deformation, limit the range of the elastic deformation, and enable the bolt as a whole to have sufficient supporting strength.

Preferably, the end head 242 is provided with a cross flower slot 245 for easy screwing of the center regulating rod 24. By screwing the cross flower slot 245, the center regulating rod 24 can be adjusted to be screwed into the depth of the circular platform 231 of the stem tail 23, thereby adjusting the distance between the shoulder body connecting piece 21 and the stem tail 23, that is, the total length of the bolt 2. The bending deformation ability of the flexible stem body 22 can also be affected. The appropriate screwing depth can be chosen according to the age and other conditions of the patient who is provided with the flexible hip joint stem.

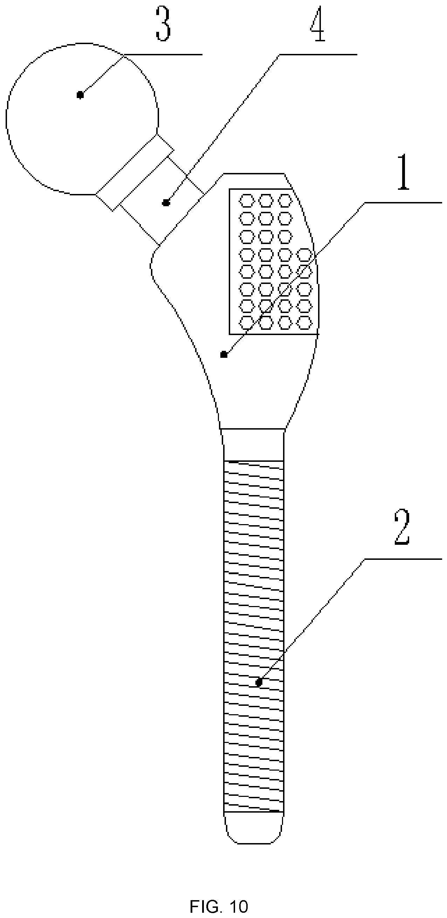

The present invention further provides flexible hip joint prosthesis. The flexible hip joint includes a femoral stem 1 and a flexible hip joint stem 2, as shown in FIG. 10, wherein the flexible hip joint can produce a bond between the femoral stem 1 and the cortex on the greater trochanter, thereby producing an interaction force. That is, there is an interaction force between the femoral stem 1 and the cortex on the greater trochanter, wherein the interaction force can be transmitted to the flexible hip joint so that forces can be transmitted between the flexible hip joint and the cortex on the greater trochanter.

The femoral stem 1 abuts the inner side of the cortex on the greater trochanter.

Preferably, the femoral stem 1 is located on the side of the flexible hip joint, and its exterior is arc-shaped, which matches the shape of the inner side of the greater trochanter cortex. It can be embedded in the arc of the greater trochanter cortex, thereby increasing the contact area between the femoral stem 1 and the greater trochanter cortex. As such, the structural form in which the femoral stem 1 and the cortex are in contact with each other is more stable, i.e., the structural form is not easy to be shaken and will not easily be deflected due to uneven force.

The greater trochanter according to the present invention is the raised area above the outer side where the human femoral neck and the femoral body are connected, wherein the femoral body is the long straight bone in the middle of the femur.

An artificial spherical end 3 is further provided on the femoral stem 1. The artificial spherical end 3 is designed according to the external dimensions of the femoral head of the human body, with its upper structure being spherical. A hole is provided at the bottom of the artificial spherical end so that the artificial spherical end is connected with the femoral stem 1 through the neck rod 4 that goes deep into the hole. The artificial spherical end may be an existing artificial spherical end or an artificial spherical end with a buffering and shock absorption function.

In a preferred embodiment, a buffer device 31 is provided inside the artificial spherical end with a buffering and shock absorption function. One end of the buffer device 31 abuts the inner wall of the artificial spherical end 3 and the other end abuts the neck rod 4, wherein the neck rod 4 is fixed on the femoral stem 1. There can be a certain degree of relative movement between the artificial spherical end 3 and the neck rod 4. When the artificial spherical end 3 is under pressure, the artificial spherical end 3 tends to move downward, thereby pressing the buffer device 31. As the artificial spherical end 3 moves downward, the buffer device 31 is gradually deformed, where the elastic force on it increases gradually, and finally the artificial spherical end 3 bounces back to its original position by the buffer device 31.

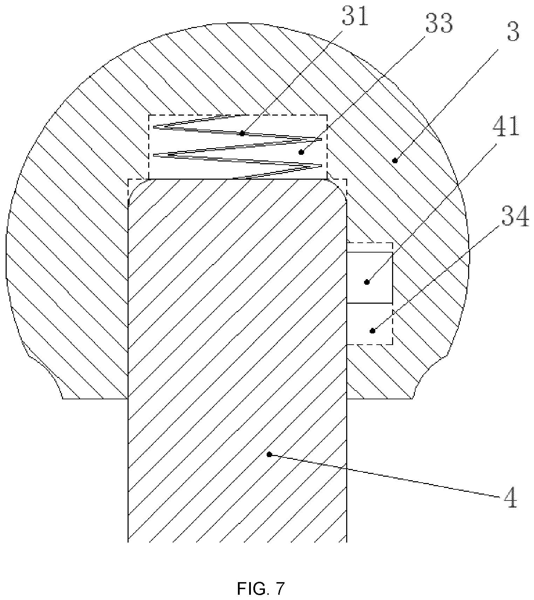

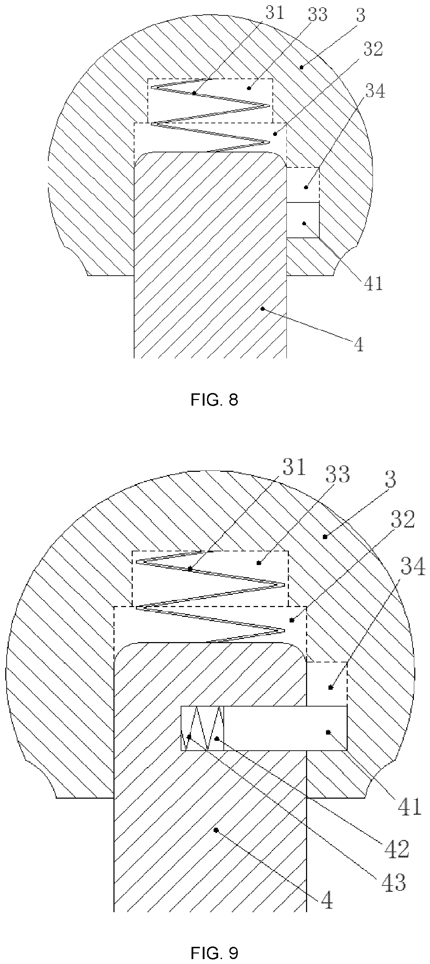

Further preferably, a neck rod hole 32 for the extension of the neck rod 4 is provided in the artificial spherical end 3, and a buffer device accommodating cavity 33 is also communicated above the neck rod hole 32, as shown in FIG. 7 and FIG. 8. The neck rod 4 can reciprocate in the neck rod hole 32. When the neck rod 4 extends into the deepest part of the neck rod hole 32, the neck rod hole 32 can be substantially filled. The hole diameter of the buffer device accommodating cavity 33 is smaller than that of the neck rod 4. As such, the neck rod 4 cannot extend into the buffer device accommodating cavity 33, thereby providing a limit in one direction for the neck rod 4. FIG. 7 illustrates the structural schematic diagram when the neck rod 4 fully extends into the neck rod hole 32. FIG. 8 illustrates the structural schematic diagram when the neck rod 4 is ejected to the maximum stroke by the buffer device 31, wherein dashed lines in the figures represent the inner contour of the artificial spherical end 3 and solid lines represent the outer contour of the artificial spherical end 3 and the neck rod 4.

Preferably, the cross-sectional dimension of the top shaft of the neck rod 4 is equal to or slightly smaller than the cross-sectional dimension of the neck rod hole 32, thereby facilitating the relative movement between the neck rod 4 and the artificial spherical end 3.

Preferably, a limit slot 34 is provided on the side wall of the accommodating cavity 33, and correspondingly, a limit protrusion 41 extending to the side is provided on the neck rod 4. The limit protrusion 41 is embedded in the limit slot 34 and moves together with the neck rod 4 in the limit slot 34. Through the cooperation between the limit protrusion 41 and the limit slot 34, a limit in another direction is provided for the movement of the neck rod 4, thereby ensuring that the neck rod 4 and the artificial spherical end 3 will not be detached from each other.

In the actual application process, the artificial spherical end 3 needs to be firmly connected with the neck rod, i.e., it cannot be detached, and it also must be ensured that the artificial spherical end 3 and the neck rod 4 can be quickly installed, reducing the difficulty of installation and saving installation time.

Preferably, a limit cavity 42 arranged laterally is provided on the neck rod 4, as shown in FIG. 9. The limit protrusion 41 is in the shape of a long rod, and most of its structure is located in the limit cavity 42. Moreover, a compressed spring 43 is provided at the bottom of the limit cavity 42. When the neck rod 4 needs to be inserted into the artificial spherical end 3, the limit protrusion 41 is pressed inwardly so that the limit protrusion 41 as a whole is embedded into the limit cavity 42, and the compressed spring 43 inside is pressed at the same time. When the limit protrusion 41 is adjacent to the limit slot 34, the compressed spring 43 will push the end of the limit protrusion 41 from the limit cavity 42 so that the limit protrusion 41 enters the limit slot 34, thereby completing the connection and installation between the neck rod 4 and the artificial spherical end 3.

The buffer device 31 may be a spring or an elastic sheet made of a biocompatible material or any other elastic mechanism, which is not particularly limited in the present application.

The artificial spherical end with a buffering and shock absorption function can improve the reliability of the connection between the artificial spherical end and the femoral stem, and can improve the service life, tensile strength, and compressive strength after connection.

In addition, the artificial spherical end with a buffering and shock absorption function can avoid a series of existing problems caused by setting the shock absorption function inside the femoral stem.

In a preferred embodiment, the femoral stem 1 can be designed with an existing femoral stem structure in the art, and the femoral stem 1 can also be configured as a detachable femoral stem.

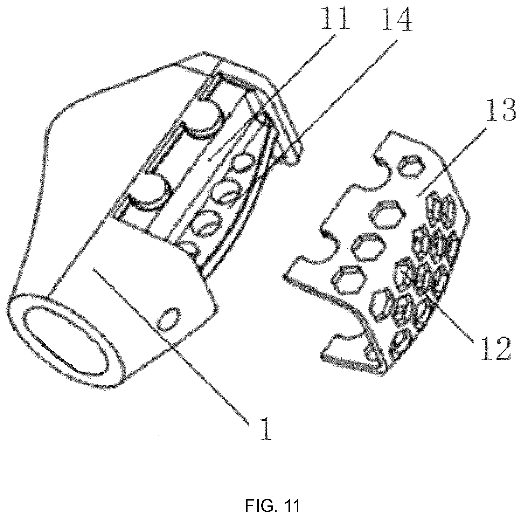

Preferably, the detachable femoral stem 1 has a reserved space 11 capable of accommodating artificial bone substitute materials on the inner side of the upper part of the femoral stem 1, as shown in FIG. 10 and FIG. 11. The surface of the femoral stem 1 is provided with a hole 12 that communicates with the reserved space 11 so that the cortex and the femoral stem 1 can naturally grow as a whole.

Preferably, the position where the reserved space is set on the femoral stem is the force-bearing portion on the femoral stem, that is, the portion that is in contact with the cortex and bears acting force.

Preferably, the surface of the force-bearing portion is in the shape of a mesh, and there are many holes 12 on it, thereby facilitating the growth of the cortex and the artificial bone in the reserved space 11 as a whole and ensuring the strength of the force-bearing portion. Preferably, the shape of the hole 12 can be any optional shape, such as a circle, an ellipse, a rectangle, a polygon, etc., which is not particularly limited in the present invention.

Further preferably, as shown in FIG. 10 and FIG. 11, the surface of the force-bearing portion is a mesh-shaped hard shell 13, which provides hard protection for the force-bearing portion and the artificial bone in it.

The hard shell 13 is detachably fixed on the femoral stem 1, thereby facilitating the implantation of artificial bone. The hard shell 13 can be disassembled depending upon the requirement so that it is detached from the femoral stem 1. As such, the reserved space becomes an open space, and the hard shell 13 is fastened on the femoral stem 1 after filling artificial bone in the open space. Consequently, the hard shell 13 is in full contact with the artificial bone on its inner side, and the interior of the reserved space 11 is filled with artificial bone, leaving no gap.

Preferably, the area of the opening on the hard shell 13 should be more than one third, more preferably about half, of the surface area of the hard shell 13.

Preferably, there can be various detachable connection relationships between the hard shell 13 and the femoral stem 1, such as a rotary connection with a rotating shaft, a snap-on connection that uses the elasticity of the material to achieve a fixed connection, or a bolt-type structure that is fixed by interference fit. In the present invention, a snap-on connection is preferred. The hard shell 13 and the femoral stem 1 are provided with snap-on structures that cooperate with each other. The hard shell 13 has certain elasticity so that it can be fixed on the femoral stem 1 and can also be detached multiple times.

In a preferred embodiment, in the force-bearing portion, a support ridge 14 topping the hard shell 13 is provided on the inner side of the hard shell 13, as shown in FIG. 10 and FIG. 11. There can be one or more support ridges 14, which are evenly distributed in the reserved space. One end of the support ridge 14 is fixed on the femoral stem 1, that is, at the bottom of the reserved space, and the other end abuts the hard shell 13 fastened on the femoral stem 1 to provide support for the hard shell 13 so as to prevent excessive compression of bone cement when the hard shell 13 is concave and deformed inwardly. Under the action of the support ridge 14, the hard shell 13 basically will not be concave and deformed inwardly.

Preferably, a through-hole is provided on the wall surface of the support ridge 14, which enables the bone materials in the two reserved spaces separated by the support ridge 14 to be connected to each other and facilitates their growth with the support ridge 14 into an integral structure in the subsequent growth.

The greater trochanter is removed in a general hip replacement surgery. Therefore, the fixation made only by the splice relationship between the bolt and the femoral medullary cavity after placement of the artificial hip joint will cause great damage to the human body, and the actual technical effect is not satisfactory. The setting of the force-bearing portion can retain the greater trochanter and the cortex on it. The cortex is attached to the force-bearing portion, and then they gradually grow into an integral part, providing a support point for the femoral stem in another direction, which greatly improves the stability between the femoral stem and the femoral body and prolongs the service life of the artificial hip joint.

### EXPERIMENTAL EXAMPLES

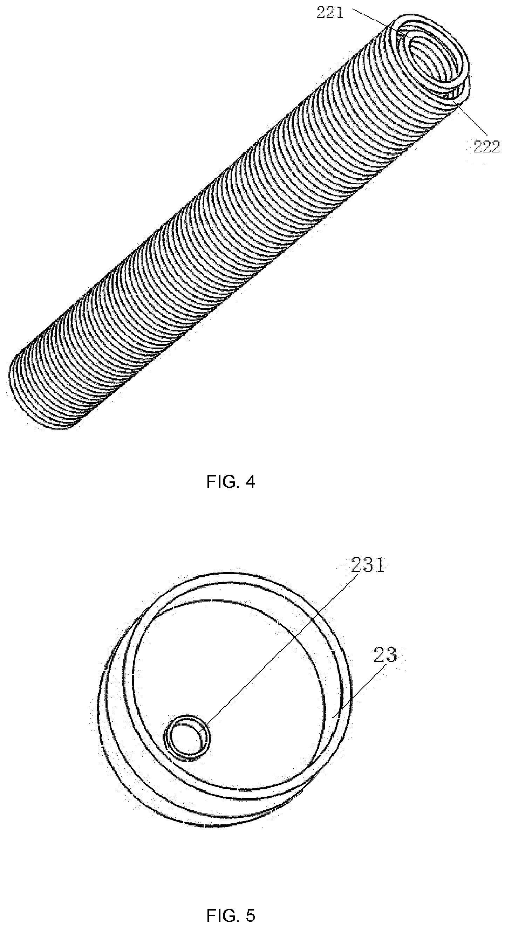

The flexible stem body includes an inner-layer cylinder and an outer-layer cylinder, as shown in FIG. 4. Both the inner-layer cylinder and the outer-layer cylinder are composed of spiral springs. In the absence of external force, any two adjacent coils of spring wires of the inner-layer cylinder are attached to each other, and any two adjacent coils of spring wires of the outer-layer cylinder are attached to each other. Moreover, both the inner-layer cylinder and the outer-layer cylinder are formed by winding the same wire. The cross-sectional shape of the wire is a rectangle, wherein the length of any of the four sides of the rectangle is 1.2 mm. The spiral spring of the inner-layer cylinder rotates anticlockwise and the spiral spring of the outer-layer cylinder rotates clockwise. There is a gap of 3 mm between the inner-layer cylinder and the outer-layer cylinder.

Experimental Example 1: the flexible stem body is subjected to a tensile fatigue test according to the experimental method in GB_T 16947-2009, wherein the flexible stem body is repeatedly stretched, and the amount of stretching is 10 mm each time. After 125,798,688 times of continuous stretching, cracks appear in the flexible stem body.

Experimental Example 2: the flexible stem body is subjected to a twist fatigue test according to the experimental method in GB_T 16947-2009, wherein the flexible stem body is repeatedly twisted. The directions of two adjacent twists are different. The twist angle for each twist is 10 degrees, i.e., an angle of 10 degrees is twisted clockwise, an angle of 10 degrees is twisted anticlockwise after returning to the original position, and then an angle of 10 degrees is further twisted clockwise after returning to the original position. After 106,583,272 times of continuous twists, cracks appear in the flexible stem body.

Experimental Example 3: the flexible stem body is subjected to a fatigue test of stretch and alternating twist according to the experimental method in GB_T 16947-2009, wherein the specific treatment process is repeated to perform fatigue test treatment on the flexible stem body. The specific treatment process includes stretching 10 mm, twisting an angle of 10 degrees clockwise after returning to the original position, and then twisting an angle of 10 degrees anticlockwise after returning to the original position. After 31,854,362 times of specific treatments, cracks appear in the flexible stem body.

According to the experimental results, the flexible stem body can withstand at least 31,854,362*3=95,563,086 twists or stretches, calculated by walking 10,000 steps per day: 95,563,086/5,000=19,112 days, 19,112/365=52 years. Theoretically, the flexible stem body according to the present application can be used for 52 years.

The present invention has been described above with reference to the preferred embodiments, which are merely exemplary and function for illustrations. On this basis, various replacements and improvements can be made to the present invention, and they all fall within the scope of protection of the present invention.

## Claims

1. A flexible hip joint stem, **characterized in that**,
the flexible hip joint stem is used for being inserted into a femoral medullary cavity on a femoral body,
the flexible hip joint stem includes a flexible stem body (22) with lateral deformation capability,
the flexible stem body (22) can be deformed in the femoral medullary cavity along physiological arc of a human femoral medullary cavity and is in close contact with the femoral medullary cavity, whereby the flexible hip joint stem is mounted on the femoral body through the cooperation of the flexible stem body (22) with the femoral medullary cavity.

2. The flexible hip joint stem according to claim 1, **characterized in that**:
the flexible stem body (22) includes an inner-layer cylinder (221) and an outer-layer cylinder (222); and
the inner-layer cylinder (221) and the outer-layer cylinder (222) are both composed of spiral springs,
in the absence of external force:
any two adjacent coils of spring wires of the inner-layer cylinder (221) are attached to each other, and
any two adjacent coils of spring wires of the outer-layer cylinder (222) are attached to each other.

3. The flexible hip joint stem according to claim 2, **characterized in that**:
the inner-layer cylinder (221) and the outer-layer cylinder (222) are formed by winding the same wire.

4. The flexible hip joint stem according to claim 3, **characterized in that**:
a cross-sectional shape of the wire is rectangular.

5. The flexible hip joint stem according to claim 2, **characterized in that**:
the spiral spring of the inner-layer cylinder (221) rotates in an opposite direction to that of the outer-layer cylinder (222).

6. The flexible hip joint stem according to claim 2, **characterized in that**:
a predetermined gap is left between the inner-layer cylinder (221) and the outer-layer cylinder (222),
preferably, a gap of 2-4 mm is left between the inner-layer cylinder (221) and the outer-layer cylinder (222).

7. The flexible hip joint stem according to claim 1, **characterized in that**:
the flexible hip joint stem further includes a shoulder body connecting piece (21), a stem tail (23), and a center regulating rod (24).

8. The flexible hip joint stem according to claim 7, **characterized in that**:
the shoulder body connecting piece (21) is mounted at a top end of the flexible stem body (22),
the stem tail (23) is in a quasi-conical shape and is mounted at a bottom end of the flexible stem body (22), and
the center regulating rod (24) passes through the shoulder body connecting piece (21) and the flexible stem body (22) and is fixed with the stem tail (23) by screwing threads.

9. The flexible hip stem according to claim 8, **characterized in that**:
a main body of the center regulating rod (24) is a long straight rod (241), a top of which is provided with an end head (242) and a bottom of which is engraved with threads (243), and
the end head (242) is provided with a cross flower slot (245) which facilitates screwing of the center regulating rod (24).

10. A flexible hip joint prosthesis, **characterized in that**:
the flexible hip joint prosthesis includes a femoral stem (1) and a flexible hip joint stem (2), wherein an artificial spherical end (3) is further provided on the femoral stem (1).
